# EUROPEAN PATENT SPECIFICATION

(11) **EP 3 158 954 B1**
(45) Date of publication and mention of the grant of the patent: **27.03.2019**
(21) Application number: 16178937.5
(22) Date of filing: 12.07.2016
(51) Int. Cl.: A61B 17/221, A61B 17/3207

(54) **CATHETER**
KATHETER
CATHÉTER

(30) Priority: 20.10.2015 JP 2015206219
(43) Date of publication of application: 26.04.2017
(73) Proprietor: Asahi Intecc Co., Ltd., Seto-shi, Aichi 489-0071 (JP)
(72) Inventor: Nishigishi, Makoto, Seto-shi, Aichi 489-0071 (JP)
(74) Representative: Winter, Brandl, Fürniss, Hübner, Röss, Kaiser, Polte - Partnerschaft mbB

(56) References cited:
- WO-A1-94/24946
- US-A- 5 180 368
- US-A1- 2002 091 409
- US-A1- 2007 225 750
- US-A1- 2011 264 132
- US-A1- 2015 005 810

## Description

### TECHNICAL FIELD

The present invention relates to a catheter for capturing a blockage in a blood vessel and/or a retrograde guide wire.

### BACKGROUND ART

Conventionally, the occlusion of a blood vessel such as chronic total occlusion (CTO) may occur due to a blockage in the blood vessel. Medical devices used for treating such an incidence include, for example, a device (catheter) for removing a blockage in a vessel as disclosed in, for example, JP 3655920 B, the device comprising a blockage-removing element in which two or more wires are interwoven so as to expand or contract in a radial direction for the purpose of removing the blockage in the vessel.

Further, for example, JP 2011-517424 A discloses a catheter having a mesh-like self-expandable area. The aforementioned catheter comprises a cover (a protective film) at the proximal end part of the self-expandable area, the cover serving as a funnel for guiding or directing a blood flow into a lumen of the catheter. Note that the above cover may be formed directly on the mesh by means of adhesive bonding, welding, coating or the like, or may be attached to the mesh by performing coupling and the like with a mechanical fastening device.

US 2015/0005810 A and US 2011/0264132 respectively disclose a catheter comprising a cover which is only partially attached to the mesh.

Further, the use of the foregoing catheter comprising a mesh-like member expandable in a radial direction is also well known in order to capture a retrograde guide wire inserted from the opposite side of a blockage in a blood vessel.

### TECHNICAL PROBLEM

However, the configuration described in JP 3655920 B has the following problem: a blockage removed by scraping with two or more expanded wires needs to be taken up into the catheter through a narrow distal end, and thus much is left behind, resulting in a poor blockage-removing efficiency.

Moreover, in the case of capturing a retrograde guide wire, the blockage-removing element is allowed to expand to raise the wires, and while maintaining this state, the retrograde guide wire is inserted through the mesh of the blockage-removing element, and then the blockage-removing element is forced to contract for allowing the wires to lie down, thereby constraining the retrograde guide wire with the two or more wires. Therefore, disadvantageously, the retrograde guide wire may forcibly be bent, resulting in a risk of breakage.

Furthermore, the configuration described in JP 2011-517424 A has the following problem: the expansion or contraction of the mesh may not be performed smoothly because there is no degree of freedom in the movement of the cover relative to that of the mesh.

WO 94/24946 A1 discloses a catheter comprising a hollow shaft with a tubular mesh member joined to a distal end of the hollow shaft, the tubular mesh member comprising at least a first wire and a second wire interwoven so as to expand and contract in a radial direction, a distal tip joined to a distal end of the tubular mesh member, a core wire joined to the distal tip, the core wire controlling an expansion and contraction of the tubular mesh member and a protective covering on the mesh.

Accordingly, an objective of the present invention is to provide a catheter in which the efficiency for removing a blockage in a blood vessel can be improved, and breakage of a retrograde guide wire can be prevented, and expansion and contraction of a mesh member having a protective film can be performed smoothly.

### SOLUTION TO PROBLEM

The present invention provides a catheter comprising: a hollow shaft; a tubular mesh member joined to a distal end of the hollow shaft, the tubular mesh member comprising at least a first wire and a second wire interwoven so as to expand and contract in a radial direction; a distal tip joined to a distal end of the mesh member; a core wire joined to the distal tip, the core wire controlling an expansion and contraction of the tubular mesh member; and a protective film covering at least a portion of the inner periphery of a proximal end part of the tubular mesh member, wherein the protective film is joined only to any one of either the first wire or the second wire.

According to the aforementioned configuration, a blockage in a blood vessel can be guided to the distal end of the catheter by virtue of the protective film, enhancing the efficiency for removing the blockage in the blood vessel. Further, a retrograde guide wire entering into the mesh member can directly and smoothly be guided into the catheter by means of the guiding effect of the protective film. Therefore, the retrograde guide wire needs not to be constrained by expansion and contraction operations of the mesh member, preventing the retrograde guide wire from breakage. Furthermore, in the mesh member to which the protective film is attached in the aforementioned manner, the first wire and the second wire are not constrained by each other via the protective film. Therefore, the mesh member can be expanded and contracted smoothly.

Moreover, a proximal end of the protective film and the distal end of the hollow shaft may be spaced from each other. However, the proximal end of the protective film preferably extends through a position of the distal end of the hollow shaft.

According to the aforementioned configuration, a blockage in a blood vessel and a retrograde guide wire can be guided into the hollow shaft smoothly with nothing left behind at the joining portion between the mesh member and the hollow shaft.

Further, it is preferred that the distal end part of the protective film is joined to any one of either the first wire or the second wire, while the proximal end part of the protective film is joined to neither the first wire or the second wire.

According to the aforementioned configuration, the distal end part of the protective film follows the mesh member to expand in a radial direction when the diameter of the mesh member is increased. In contract, the proximal end part of the protective film does not follow the movement of the mesh member, and thus does not expand in a radial direction. Therefore, according to the aforementioned configuration, the protective film may take a funnel-like shape with a diameter decreased toward a direction of a proximal end of the protective film when the tubular mesh member is expanded (that is, when the diameter of the mesh member is increased). Moreover, the proximal end part of the protective film is joined to neither the first wire nor the second wire of the mesh member. Therefore, the flexibility of the catheter in a blood vessel will not be impaired.

Furthermore, the protective film may be joined to any one of either the first wire or the second wire from a distal end part of the protective film through the proximal end part thereof. Also, the catheter may have a dual-lumen structure.

### ADVANTAGEOUS EFFECTS OF THE INVENTION

The catheter according to the present invention can provide the following advantageous effects: the efficiency for removing a blockage in a blood vessel can be enhanced; breakage of a retrograde guide wire can be prevented; and expansion and contraction operations of the mesh member having the protective film can be performed smoothly.

### BRIEF DESCRIPTION OF THE DRAWINGS

Figs. 1A and 1B show the catheter according to Example 1 in a state where the mesh member is contracted: Fig. 1A is a schematic drawing of an overview; and Fig. 1B is a cross sectional view along A-A in Fig. 1A.
Fig. 2 is a schematic view illustrating the catheter according to Example 1 in a state where the mesh member is expanded.
Fig. 3 is an enlarged partial cross-sectional view showing a manner of joining between the mesh member and the protective film according to Example 1 in which the cross sectional view along B-B in Fig. 1A is enlarged.
Fig. 4 is a schematic view illustrating the catheter according to Example 1 in a state of use where a retrograde guide wire is inserted through.
Fig. 5 is a schematic view illustrating the catheter according to Example 2.
Fig. 6 is a schematic view illustrating the catheter according to Example 3.
Fig. 7A and 7B show the catheter according to Example 4: Fig. 7A is a schematic drawing of an overview; and Fig. 7B is a cross sectional view along C-C in Fig. 7A.

### DESCRIPTION OF EMBODIMENTS

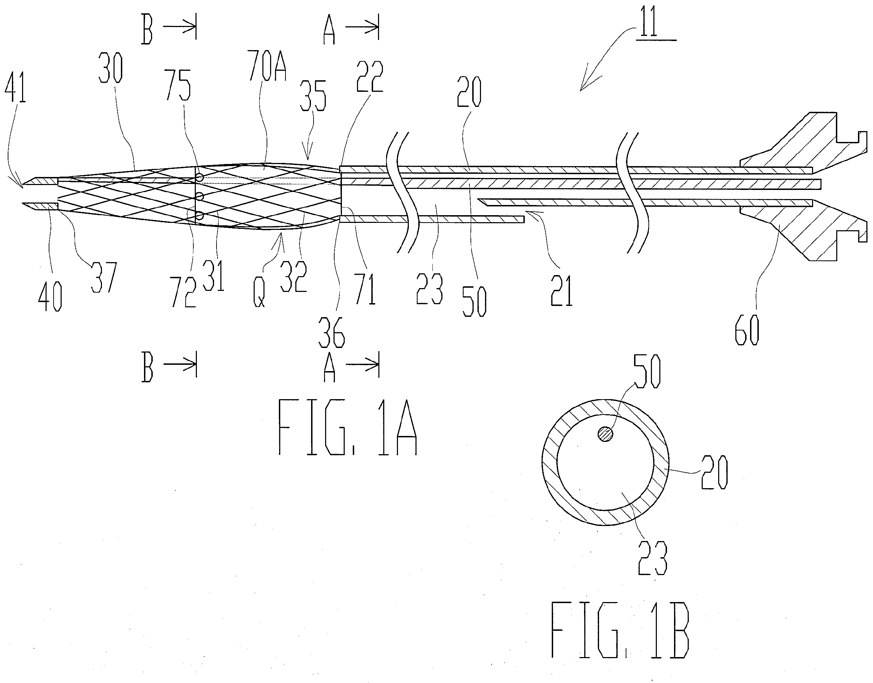

Below, Examples in which the catheters according to the present invention are embodied will be described in detail. However, the present invention shall not be limited to Examples shown below, and modifications in design can be made appropriately. Note that in Fig. 1A, Fig. 2 and Figs. 4 to 7A, the left side corresponds to the distal end side which is to be inserted into the body, and the right side corresponds to the proximal end side which is to be operated by an operator such as a physician.

### Example 1

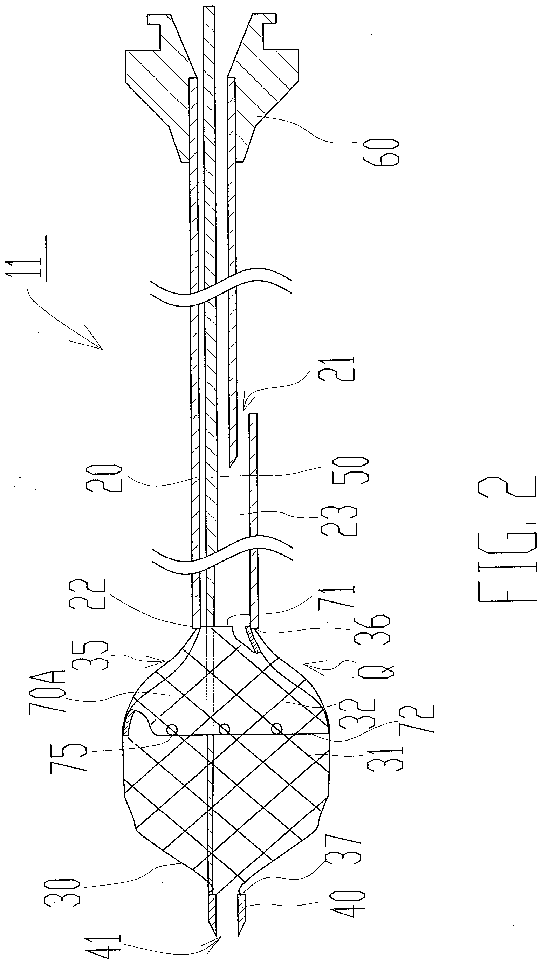

For example, a catheter 11 used for removing a blockage in a blood vessel comprises a hollow shaft 20 as shown in Figs. 1A, 1B and 2.

The hollow shaft 20 forms a hollow tubular shape so as to have a lumen 23 therein, in which a RX (rapid exchange) port 21 opened toward to the proximal end side is formed at a side wall between the distal end part and the proximal end part.

Note that the hollow shaft 20 is preferably configured with a tube comprising, for example, a resin such as polyamide, polyamide elastomer, polyolefine, polyester or polyester elastomer. Further, a material of the proximal end side may be different from that of the distal end side. For example, the proximal end side may comprise a superelastic alloy such as stainless steel (SUS 304) and a Ni-Ti alloy.

Moreover, a mesh member 30 is joined to a distal end 22 of the hollow shaft 20. More specifically, the mesh member 30 has a tubular shape in which a first wire 31 and a second wire 32 are interwoven, and a proximal end 36 of the mesh member 30 is joined to a distal end 22 of the hollow shaft 20 such that the mesh member 30 is substantially coaxially aligned with the hollow shaft 20.

Note that the first wire 31 and the second wire 32 of the mesh member 30 preferably comprise, for example, a metal material such as stainless steel (SUS 304). In particular, an operator can accurately detect the location of the mesh member 30 during coronary angiography in a case where a radiopaque material (for example, tungsten or a Co-Cr alloy) is used in at least one of either the first wire 31 or the second wire 32.

Further, a hollow tubular distal tip 40 is joined to a distal end 37 of the mesh member 30. More specifically, the distal tip 40 has a tubular shape, and an opening 41 is formed at the distal end of the distal tip 40.

Note that the distal tip 40 is desirably formed with a soft resin such as polyurethane or polyurethane elastomer.

Further, the distal end of a core wire 50 is joined to the proximal end of the distal tip 40. More specifically, the core wire 50 is inserted through the inside of the hollow shaft 20 (the lumen 23), and is configured with a tapered metal wire material having a circular cross section and having a diameter decreased toward the distal end. As described below, the mesh member 30 can be expanded or contracted in a radial direction by controlling and operating the core wire 50 in an axial direction.

Further, a connector 60 is joined to the proximal end of the hollow shaft 20.

Furthermore, a thin flexible protective film 70A is arranged in a tube-like fashion at the inner periphery of the proximal end side 35 of the mesh member 30. Note that for easier understanding, the hollow shaft 20, the distal tip 40 and the connector 60 as well as the core wire 50 disposed inside the hollow shaft 20 and the mesh member 30 are shown in a cross sectional view in Figs. 1A and 2. Note that a portion of the core wire 50 inside the protective film 70A is shown with dashed lines.

Note that the protective film 70A is desirably formed with, for example, a resin such as polyethylene, polyurethane, polyamide, polyamide elastomer, polyolefine, polyester or polyester elastomer, and a material used for a balloon of a common balloon catheter can suitably be used.

Below, the relevant parts of the present invention will be described. The protective film 70A covers the entire inner periphery of the proximal end side 35 of the mesh member 30. Further, a proximal end 71 of the protective film 70A extends through a position of the distal end 22 of the hollow shaft 20, and is positioned inside the hollow shaft 20 such that the proximal end 71 of the protective film 70A is not joined to the distal end 22 of the hollow shaft 20.

Moreover, only the distal end part of the protective film 70A is joined to the mesh member 30 with an adhesive 75, and the portion of the protective film 70A except for the distal end part serves as a non-joining portion Q which is not joined to the mesh member 30 at any position.

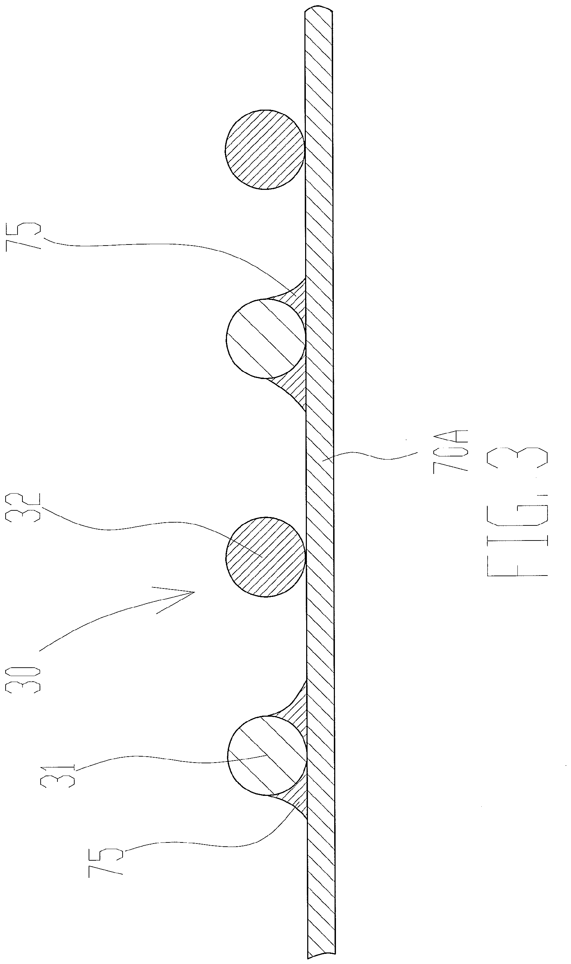

As shown in Fig. 3 in more detail, the distal end part of the protective film 70A is joined to the first wire 31 of the mesh member 30 via the adhesive 75, but not to the second wire 32 which intersects with the first wire 31. Therefore, the protective film 70A and the second wire 32 are allowed to independently move relative to each other without being constrained by each other.

Next, aspects of the use of the foregoing catheter 11 will be described.

According to the catheter 11, the mesh member 30 becomes expanded in the diameter (see Fig. 2) or contracted in the diameter (see Fig. 1A) when the core wire 50 is controlled and operated in an axial direction to change the relative distance between the distal tip 40 and the hollow shaft 20.

Then, the catheter 11 is to be inserted into a blood vessel in a state where the diameter of the mesh member 30 is reduced as shown in Fig. 1A. Subsequently, for example, when the mesh member 30 reaches a predetermined position such as a CTO site, the core wire 50 is operated so that the diameter of the mesh member 30 becomes increased as shown in Fig. 2. Note that a position where the diameter of the mesh member 30 is increased may appropriately be changed according to situations.

As the diameter of the mesh member 30 is increased as described above, the distal end side of the protective film 70A follows the change in the state of the mesh member 30, and the diameter thereof is increased, so that the entire protective film 70A becomes funnel-like (see Fig. 2)

In a state where the diameter of the mesh member 30 is increased as described above, a blockage and the like can be taken up into the hollow shaft 20 through the distal end 72 of the protective film 70A.

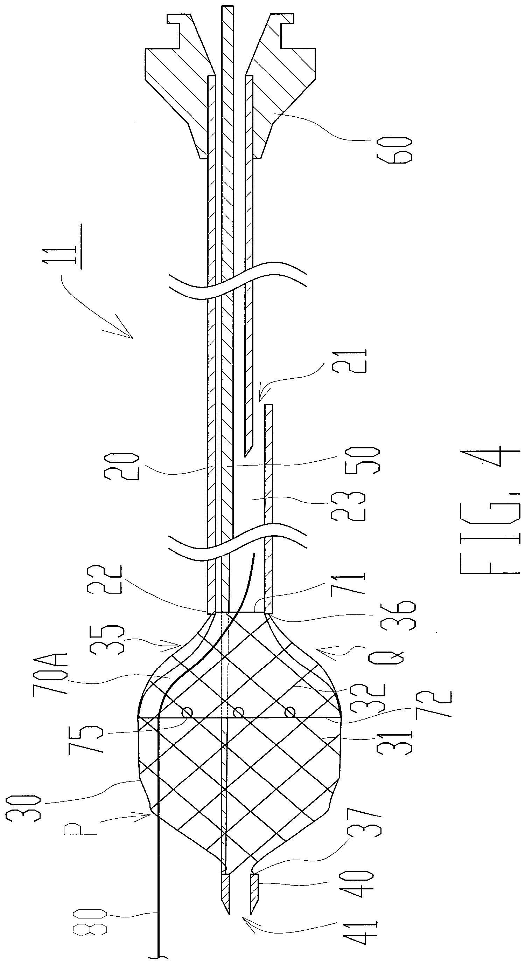

Further, in a case where a retrograde guide wire 80 is inserted from the opposite side of the aforementioned catheter 11, the distal end part of the mesh member 30 in a state where the diameter thereof is increased serves as an introducing portion P through which the retrograde guide wire 80 is to be introduced, and the retrograde guide wire 80 is then inserted into the mesh member 30 through the introducing portion P as shown in Fig. 4. Then, the retrograde guide wire 80 inserted into the mesh member 30 is guided into the hollow shaft 20 by virtue of the guiding effect of the protective film 70A which has taken a funnel-like shape. Subsequently, the retrograde guide wire 80 is removed from the body through the lumen 23 and the RX port 21 of the hollow shaft 20. By this, the retrograde guide wire 80 which has been guided into the catheter 11 can be retrieved without breakage. As described above, the catheter 11 according to this Example 1 can suitably be used as a medical device in combination with the retrograde guide wire 80.

Here, the distal end part of the protective film 70A in this Example 1 has a high degree of freedom in expanding the diameter of the mesh member 30 because it is joined only to the first wire 31 of the mesh member 30. That is, when the diameter of the mesh member 30 is increased, the distal end part of the protective film 70A follows only the movement of the first wire 31 of the mesh member 30, and expands in a radial direction. This can avoid the problem of the mesh member 30 that the first wire 31 and the second wire 32 are otherwise mutually restricted by the distal end part of the protective film 70A. Therefore, the mesh member 30 can be expanded or contracted smoothly.

Further, the proximal end part of the protective film 70A is not expanded in a radial direction even when the mesh member 30 is expanded because it is not joined to the first wire 31 and the second wire 32. Therefore, the protective film 70A when increasing the diameter of the mesh member 30 can easily be made into a funnel like shape with a diameter decreased toward the direction of the proximal end. Moreover, the flexibility of the catheter 11 in a blood vessel is not impaired because the proximal end part of the protective film 70A is joined to neither the first wire 31 nor the second wire 32 of the mesh member 30 as described above.

Furthermore, the proximal end 71 of the protective film 70A extends to a position of the distal end 22 of the hollow shaft 20 as described above. Therefore, a blockage in a blood vessel and a retrograde guide wire 80 can be guided into the hollow shaft 20 smoothly with nothing left behind at the joining portion between the mesh member 30 and the hollow shaft 20. Note that the proximal end 71 of the protective film 70A may further extend to a position of the proximal end side relative to the distal end 22 of the hollow shaft 20 (in other words, through the lumen 23 of the hollow shaft 20).

### Example 2

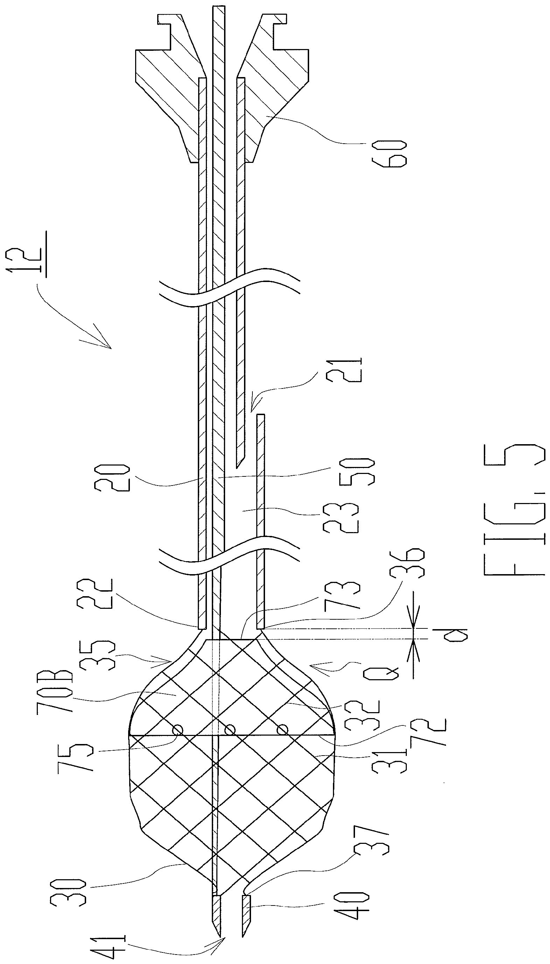

A catheter 12 according to Example 2 will be described with reference to Fig. 5. Note that the same reference numbers are assigned to structures similar to those in Example 1 above, and the descriptions thereof are omitted.

According to the catheter 12, a proximal end 73 of a protective film 70B and the distal end 22 of the hollow shaft 20 are spaced by a distance, d, and the protective film 70B partially covers the inner periphery of the proximal end side of the mesh member 30. In the aforementioned configuration, the proximal end 73 of the protective film 70B is not constrained by the hollow shaft 20. Therefore, the degree of freedom in expanding or contracting the protective film 70B is further improved, leading to even smoother operations of expanding or contracting the mesh member 30.

### Example 3

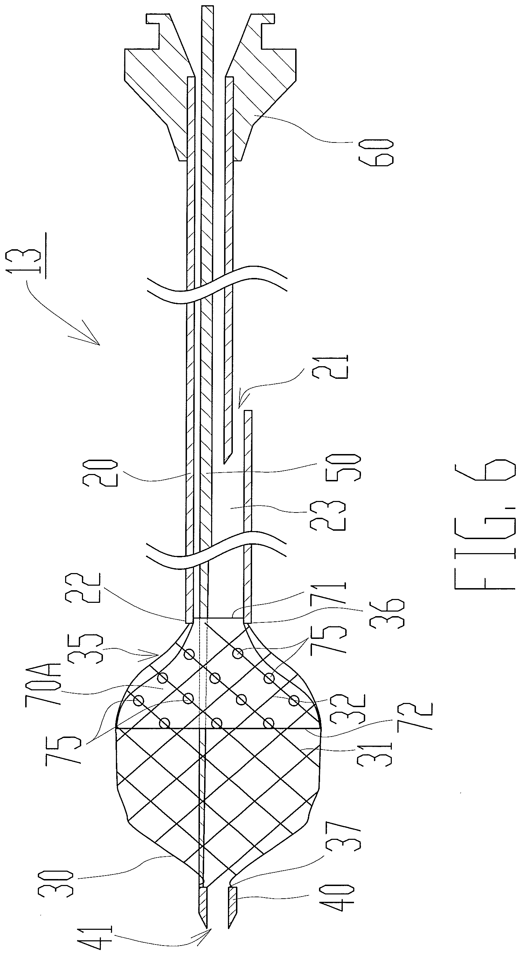

A catheter 13 according to Example 3 will be described with reference to Fig. 6. Note that the same reference numbers are assigned to structures similar to those in Example 1 above, and the descriptions thereof are omitted.

The protective film 70A of the catheter 13 is joined only to the first wire 31 of the mesh member 30 via the adhesive 75 from the distal end part through the proximal end part of the protective film 70A. That is, the catheter 13 does not substantially comprise the aforementioned non-joining portion Q. Again in the aforementioned configuration, the protective film 70A is joined only to the first wire 31 of the mesh member 30. Therefore, the mesh member 30 can smoothly be expanded or contracted.

### Example 4

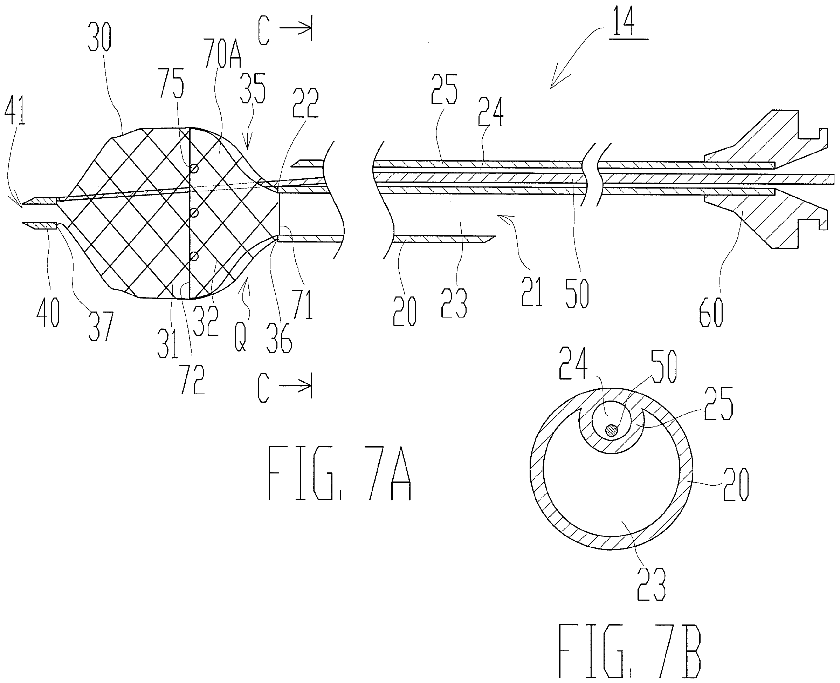

A catheter 14 according to Example 4 will be described with reference to Fig. 7A and 7B. Note that the same reference numbers are assigned to structures similar to those in Example 1 above, and the descriptions thereof are omitted.

The catheter 14 has a so-called dual-lumen structure in which a second hollow shaft 25 distinct from the hollow shaft 20 is inserted into the hollow shaft 20, and the hollow shaft 20 and the second hollow shaft 25 are fused each other. The lumen 23 is formed between the hollow shaft 20 and the second hollow shaft 25, and a second lumen 24, through which the core wire 50 is inserted, is formed inside the second hollow shaft. In the aforementioned configuration, unlike the catheters 11, 12 and 13 according to Examples 1 to 3, the core wire 50 is not inserted into the lumen 23 of the hollow shaft 20. Therefore, interference between the core wire 50 and the retrograde guide wire 80 can suitably be avoided in the lumen 23 of the hollow shaft 20. Further, since a blockage and the like taken up into the lumen 23 of the hollow shaft 20 does not adhere to the core wire 50, an operation of the core wire 50 in an axial direction is not blocked by the blockage and the like, and the mesh member 30 can smoothly be expanded or contracted.

Note that modifications can appropriately be made to the present invention in addition to the aforementioned Examples 1 to 4. For example, the hollow shaft 20 may have a mono-layer structure, or may have a multi-layer structure with two or more layers.

Further, the mesh member 30 is not limited to those comprising only the first wire 31 and the second wire 32, but may be those further comprising an additional wire.

Moreover, the catheters 11, 12, 13, 14 are not limited to configurations in which the protective film 70A, 70B and the first wire 31 are joined via the adhesive 75. For example, it may have a configuration in which the protective film 70A is partially fused and joined to the first wire 31. Furthermore, there is no particular limited for joining means for a joining portion between corresponding members in the catheter 11, 12, 13, 14.

Further, the protective film 70A, 70B may be joined only to the second wire 32 instead of the first wire 31. Note that in Examples 1 to 4, the protective film 70A, 70B and the first wire 31 are joined at a position off an intersection between the first wire 31 and the second wire 32, but the configuration is not limited to this. The protective film 70A, 70B may be joined only to any one of either the first wire 31 or the second wire 32 at an intersection between the first wire 31 and the second wire 32.

Further, the protective film 70A, 70B may be configured with, for example, a mesh material and the like finer than the mesh member 30 as long as it can guide a blockage in a blood vessel and the retrograde guide wire 80 into the hollow shaft 20.

Moreover, off course, a plurality of the protective films 70A, 70B may be arranged along the axial direction against the mesh member 30.

Note that, for the retrograde guide wire 80, those commonly used and publicly known can suitably be used.

### DESCRIPTION OF SYMBOLS

- 11, 12, 13, 14: Catheter
- 20: Hollow shaft
- 22: Distal end
- 25: Core shaft
- 30: Mesh member
- 31: First wire
- 32: Second wire
- 35: Proximal end part
- 40: Distal tip
- 50: Core wire
- 70A, 70B: Protective film
- 71: Proximal end

## Claims

1. A catheter (11, 12, 13, 14) comprising:
a hollow shaft (20);
a tubular mesh member (30) joined to a distal end (22) of the hollow shaft (20), the tubular mesh member (30) comprising at least a first wire (31) and a second wire (32) interwoven so as to expand and contract in a radial direction;
a distal tip (40) joined to a distal end (37) of the tubular mesh member (30);
a core wire (50) joined to the distal tip (40), the core wire (50) controlling an expansion and contraction of the tubular mesh member (30); **characterised by**
a protective film (70A, 70B) covering at least a portion of the inner periphery of a proximal end part (35) of the tubular mesh member (30),
wherein the protective film (70A, 70B) is joined only to any one of either the first wire (31) or the second wire (32).

2. The catheter (11, 13, 14) according to claim 1, wherein a proximal end (71) of the protective film (70A) extends to a position of the distal end (22) of the hollow shaft (20).

3. The catheter (12) according to claims 1 or 2, wherein a proximal end (73) of the protective film (70B) and the distal end (22) of the hollow shaft (20) are spaced from each other.

4. The catheter (11, 12, 14) according to any one of claims 1 to 3, wherein a distal end part of the protective film (70A, 70B) is joined to any one of either the first wire (31) or the second wire (32), while a proximal end part of the protective film (70A, 70B) is joined to neither the first wire (31) nor the second wire (32).

5. The catheter (13) according to any one of claims 1 to 3, wherein the protective film (70A) is joined to any one of either the first wire (31) or the second wire (32) from a distal end part of the protective film (70A) through the proximal end part thereof.

6. The catheter (11, 12, 13, 14) according to any one of claims 1 to 5, wherein the protective film (70A, 70B) takes a funnel-like shape with a diameter decreased toward a direction of a proximal end (71,73) of the protective film (70A, 70B) when the tubular mesh member (30) is expanded.

7. The catheter (14) according to any one of claims 1 to 6, wherein the catheter (14) has a dual-lumen structure.

## Patentansprüche

1. Katheter (11, 12, 13, 14) mit:
einem Hohlschaft (20);
einem an ein distales Ende (22) des Hohlschafts (20) angefügten rohrförmigen Maschenelement (30), das für eine Expansion und Kontraktion wenigstens einen ersten Draht (31) und einen zweiten Draht (32) aufweist, die miteinander verflochten sind;
einer an ein distales Ende (37) des rohrförmigen Maschenelements (30) angefügten distalen Spitze (40);
einem an die distale Spitze (40) angefügten Kerndraht (50), der die Expansion und Kontraktion des rohrförmigen Maschenelements (30) steuert; **gekennzeichnet durch**
einen Schutzfilm (70A, 70B), der den Innenumfang eines proximalen Endabschnitts (35) des rohrförmigen Maschenelements (30) wenigstens teilweise überzieht,
wobei der Schutzfilm (70A, 70B) entweder an dem ersten Draht (31) oder an dem zweiten Draht (32) angefügt ist.

2. Katheter (11, 13, 14) nach Anspruch 1, wobei sich ein proximales Ende (71) des Schutzfilms (70A) bis zur Stelle des distalen Endes (22) des Hohlschafts (20) erstreckt.

3. Katheter (12) nach Anspruch 1 oder 2, wobei ein proximales Ende (73) des Schutzfilms (70B) und das distale Ende (22) des Hohlschafts (20) voneinander beabstandet sind.

4. Katheter (11, 12, 14) nach einem der Ansprüche 1 bis 3, wobei ein distaler Endabschnitt des Schutzfilms (70A, 70B) entweder an dem ersten Draht (31) oder an dem zweiten Draht (32) angefügt ist, während ein proximaler Endabschnitt des Schutzfilms (70A, 70B) weder an dem ersten Draht (31) noch an dem zweiten Draht (32) angefügt ist.

5. Katheter (13) nach einem der Ansprüche 1 bis 3, wobei der Schutzfilm (70A) von seinem distalen Endabschnitt bis zu seinem proximalen Endabschnitt entweder an dem ersten Draht (31) oder an dem zweiten Draht (32) angefügt ist.

6. Katheter (11, 12, 13, 14) nach einem der Ansprüche 1 bis 5, wobei der Schutzfilm (70A, 70B) eine trichterartige Form mit einem in Richtung eines proximalen Endes (71,73) des Schutzfilms (70A, 70B) abnehmenden Durchmesser hat, wenn das rohrförmige Maschenelement (30) expandiert ist.

7. Katheter (14) nach einem der Ansprüche 1 bis 6, wobei der Katheter (14) eine Dual-Lumen-Struktur hat.

## Revendications

1. Cathéter (11, 12, 13, 14) comprenant :
une tige creuse (20) ;
un élément filet tubulaire (30) relié à une extrémité distale (22) de la tige creuse (20), l'élément filet tubulaire (30) comprenant au moins un premier fil (31) et un second fil (32) entrelacés de manière à se dilater et à se contracter dans une direction radiale ;
une pointe distale (40) reliée à une extrémité distale (37) de l'élément filet tubulaire (30) ;
un fil central (50) relié à l'extrémité distale (40), le fil central (50) commandant une dilatation et une contraction de l'élément filet tubulaire (30) ; **caractérisé par**
un film protecteur (70A, 70B) recouvrant au moins une portion de la périphérie intérieure d'une partie d'extrémité proximale (35) de l'élément filet tubulaire (30),
dans lequel le film protecteur (70A, 70B), est relié seulement à l'un quelconque du premier fil (31) ou du second fil (32).

2. Cathéter (11, 13, 14) selon la revendication 1, dans lequel une extrémité proximale (71) du film protecteur (70A) s'étend jusqu'à une position de l'extrémité distale (22) de la tige creuse (20).

3. Cathéter (12) selon les revendications 1 ou 2, dans lequel une extrémité proximale (73) du film protecteur (70B) et l'extrémité distale (22) de la tige creuse (20) sont espacées l'une de l'autre.

4. Cathéter (11, 12, 14) selon l'une quelconque des revendications 1 à 3, dans lequel une partie d'extrémité distale du film protecteur (70A, 70B) est reliée à l'un quelconque du premier fil (31) ou du second fil (32), alors qu'une partie d'extrémité proximale du film protecteur (70A, 70B) n'est reliée ni au premier fil (31) ni au second fil (32).

5. Cathéter (13) selon l'une quelconque des revendications 1 à 3, dans lequel le film protecteur (70A) est relié à l'un quelconque du premier fil (31) ou du second fil (32) depuis une partie d'extrémité distale du film protecteur (70A) par le biais de la partie d'extrémité proximale de celui-ci.

6. Cathéter (11, 12, 13, 14) selon l'une quelconque des revendications 1 à 5, dans lequel le film protecteur (70A, 70B) prend une forme d'entonnoir ayant un diamètre diminuant vers une direction d'une extrémité proximale (71, 73) du film protecteur (70A, 70B) quand l'élément filet tubulaire (30) est dilaté.

7. Cathéter (14) selon l'une quelconque des revendications 1 à 6, dans lequel le cathéter (14) présente une structure à deux lumières.
